# FASCICULE DE BREVET EUROPEEN

(11) **EP 1 259 271 B1**
(45) Date de publication et mention de la délivrance du brevet: **31.10.2007**
(21) Numéro de dépôt: 01909907.6
(22) Date de dépôt: 27.02.2001
(51) Int. Cl.: A61L 27/46, A61L 27/12

(54) **PROCEDE DE PREPARATION D'UN MATERIAU PATEUX PHOSPHOCALCIQUE INJECTABLE**
VERFAHREN ZUR HERSTELLUNG EINES PASTÖSEN, INJIZIERBAREN KALZIUMPHOSPHATMATERIALS
METHOD FOR PREPARING A CALCIUM PHOSPHATE PASTY MATERIAL FOR INJECTION

(30) Priorité: 01.03.2000 FR 0002615
(43) Date de publication de la demande: 27.11.2002
(73) Titulaire: Ceravic, 65500 Vic en Bigorre (FR)
(72) Inventeur: LACOUT, Jean-Louis, F-31000 Toulouse (FR); FRECHE, Michèle, F-31130 Fonsegrives-Quint (FR); GONCALVES, Stéphane, 31240 L'Union (FR); RODRIGUEZ, Fernand, F-31320 Aureville (FR)
(74) Mandataire: Morelle, Guy Georges Alain
(86) Numéro de dépôt international: PCT/FR2001/000563
(87) Numéro de publication internationale: WO 2001/064260

(56) Documents cités:
- EP-A- 0 276 836
- EP-A- 0 684 046
- DATABASE WPI Week 28, 1987 Derwent Publications Ltd., London, GB; AN 1987-194482 XP002154942 & JP 62 122670 A (NIPPON STEEL), 3 juin 1987 (1987-06-03)

## Description

L'invention concerne un procédé de préparation d'un matériau pâteux injectable à partir d'un mélange d'eau et de phosphates calciques, apte à évoluer, durcir et former une hydroxyapatite constituant un biomatériau utilisable en particulier en orthopédie ou art dentaire. Par matériau pâteux "injectable", on entend la faculté du matériau pâteux de circuler dans un conduit adapté à l'application envisagée sous une pression non destructive dudit matériau et compatible avec cette application et les équipements utilisés. Par "biomatériau", on entend dans la présente description le matériau solide obtenu après durcissement, ce matériau ayant des propriétés de biocompatibilité et étant destiné à remplacer ou traiter chez l'homme ou l'animal un organe ou une fonction.

Les hydroxyapatites phosphocalciques sont des matériaux bien connus de plus en plus utilisés dans le domaine chirurgical ou dentaire en raison de leurs propriétés de biocompatibilité et d'ostéoconduction. Elles peuvent être utilisées en art dentaire pour le comblement parodontal, la restauration des crêtes osseuses, le comblement des kystes ou alvéoles après extraction dentaire... et, en chirurgie osseuse pour le comblement de défaut osseux, le comblement interstitiel entre prothèse et os cortical, l'injection dans les corps vertébraux, le traitement de l'ostéoporose... Le biomatériau ainsi mis en place peut éventuellement contenir des substances actives qui, après durcissement in situ, sont lentement diffusées.

Ces biomatériaux apatitiques sont en particulier obtenus par durcissement d'un mélange pâteux préparé par gâchage d'un mélange de phosphates calciques avec de l'eau ; dans les applications précitées, la prise et le durcissement du mélange pâteux s'effectuent in situ sur le site d'application. Actuellement, les mélanges pâteux de ce type sont soit mis en place sur des sites ouverts où ils sont appliqués manuellement ou à la spatule, soit poussés sur de très courtes distances sous forte pression sur des sites directement accessibles. Ces mélanges pâteux sont en effet inaptes à circuler sous pression modérée sur des distances supérieures à quelques centimètres en raison de leurs caractéristiques de viscosité.

Ce caractère non injectable limite le domaine d'application de ces matériaux à des interventions sur site directement accessible, qui ne représentent pas le cas général ou imposent une chirurgie ouverte, traumatisante et lourde.

La présente invention se propose de fournir un procédé de préparation d'un nouveau matériau pâteux phosphocalcique injectable, c'est-à-dire susceptible, avant son évolution, d'être amené à circuler sous des pressions modérées et sur des distances notables (quelques dizaines de centimètres), ledit matériau présentant des temps de prise et de durcissement similaires à ceux des matériaux phosphocalciques existants et conduisant, après durcissement, à un biomatériau apatitique de caractéristiques mécaniques comparables ou supérieures à celles des biomatériaux apatitiques obtenus à partir des mélanges existants.

Le procédé de préparation du matériau pâteux injectable visé par l'invention est du type dans lequel on réalise à partir d'eau et de phosphates calciques un mélange pâteux apte à évoluer, durcir et former une hydroxyapatite. Selon la présente invention, ce procédé est caractérisé en ce qu'on ajoute aux phosphates calciques ou au mélange pâteux avant injection, un méthicone en proportion pondérale par rapport au mélange supérieure à 0,3 % et inférieure à 10 %, et avantageusement sensiblement comprise entre 0,5 % et 1,4 %. Il convient de rappeler que le méthicone est un polysiloxane (de la famille des silicones) ayant une fonction CH₃ sur au moins une des liaisons du silicium de son motif.

Les expérimentations ont montré que le matériau pâteux ainsi obtenu est injectable et peut être transporté sous forme pâteuse dans des conduits de type cathéter, en particulier conduits souples, sous des pressions modérées (pression relative inférieure à 1 bar) qui sont compatibles avec les conditions d'intervention chirurgicale ou dentaire et les équipements utilisés. Ce matériau pâteux fait prise même en milieu humide et durcit de façon similaire au mélange ne contenant pas de méthicone ; toutefois en l'absence de méthicone, le mélange est impossible à injecter comme cela est connu de l'homme du métier. Au surplus les essais ont permis de constater que l'addition de méthicone était susceptible de conduire à une amélioration significative des caractéristiques mécaniques du biomatériau obtenu après durcissement.

Il faut souligner que d'une façon générale, les silicones sont des lubrifiants bien connus qui sont notamment utilisés pour revêtir des parois afin de permettre un meilleur glissement d'un solide ou d'un liquide le long de celles-ci. Dans le cas présent, le méthicone est, au contraire, intimement mélangé au matériau pâteux et ses propriétés de lubrifiant de paroi n'expliquent pas la propriété d'injectabilité obtenue pour la pâte sans dégradation des caractéristiques mécaniques du matériau après durcissement, et même avec une amélioration de celles-ci. Comme on le verra dans les exemples, la lubrification de paroi améliore légèrement la faculté de déplacement de la pâte sur quelques centimètres mais ne permet pas le transport sur quelques dizaines de centimètres sous des pressions modérées (notamment pression relative inférieure à 1 bar). La propriété d'injectabilité obtenue est difficile à expliquer à l'heure actuelle : il est probable qu'elle met en jeu un effet de glissement des particules, plaquettes et aiguilles qui se forment et évoluent au cours de la prise, glissement interparticulaire conditionné par des modifications d'interfaces dues au méthicone ; il est à noter toutefois que l'amélioration des propriétés mécaniques obtenues pour le biomatériau après durcissement laisse à penser que le méthicone a également une action physico-chimique sur l'évolution du matériau et sa cristallisation en apatite.

De préférence, comme connu en soi, on réalise un mélange pâteux de phosphates calciques ayant un rapport atomique Ca/P compris entre 1,5 et 1,67. On obtient ainsi, après injection et durcissement, un biomatériau constitué par une phase pure d'hydroxyapatite dont la composition chimique est très voisine de celle de la partie minérale de l'os. En dehors de cette plage de rapport atomique, le biomatériau obtenu est multiphasé (ce qui peut être recherché dans certaines applications).

Il est possible d'ajouter au matériau pâteux phosphocalcique conforme à l'invention des additifs, en particulier additifs connus destinés à accroître la régularité de prise de la pâte (bonne homogénéité, absence de grumeaux). Par exemple, un glycérophosphate soluble dans l'eau, notamment du glycérophosphate de sodium, de potassium, ou de calcium, peut être ajouté au mélange de sorte que le pourcentage pondéral de ce composé rapporté au mélange final soit inférieur à 10 %. Ce composé contribue à une amélioration de la régularité de prise et diminue légèrement la vitesse de prise. A noter que le méthicone participe déjà à améliorer grandement la régularité de prise de la pâte et son homogénéité de sorte que la quantité de glycérophosphates peut être inférieure à celle prévue pour des mélanges similaires ne contenant pas de méthicone. Il convient de souligner que le glycérophosphate dans ce type de réaction à froid n'est pas décomposé et ne participe pas en tant que réactif chimique à la réaction de formation de l'apatite ; les rapports atomiques Ca/P sont donc donnés dans tout le texte hors glycérophosphate.

De préférence on utilise un méthicone de viscosité sensiblement comprise entre 0,02 mPas (20 centistokes) et 2:0,5 mPas (500 centistokes) correspondant à une densité sensiblement comprise entre 0,90 et 0,98 (rapport de la masse volumique à celle de l'eau). Un diméthicone comportant deux groupements, CH₃ sur le silicium de son motif est avantageusement utilisé, en particulier un diméthicone cyclique.

Les conditions de mise en oeuvre du procédé de l'invention peuvent avantageusement être celles définies dans la demande de brevet français n° 2 776 282. Selon un mode de mise en oeuvre préféré, le mélange pâteux est en particulier réalisé à froid au moyen d'un ciment pulvérulent de phosphate tricalcique et phosphate tétracalcique, et d'une solution aqueuse contenant du calcium et du phosphate, le ciment pulvérulent et la solution aqueuse étant mélangés à température ambiante (c'est-à-dire entre environ 15° C et 30° C) ; les modalités de mise en oeuvre sont de préférence les suivantes :
- préparation du ciment pulvérulent par mélange de poudres de phosphate tricalcique, de phosphate tétracalcique et de glycérophosphate,
- préparation d'une solution aqueuse d'acide phosphorique et de chaux,
- mélange de ladite solution aqueuse et dudit ciment pulvérulent de façon que le rapport pondéral global liquide/solide L/S soit compris entre 0,30 et 0,65, afin d'obtenir une pâte homogène de rapport atomique global Ca/P compris entre 1,50 et 1,67.

Cette mise en oeuvre conduit à une bonne reproductibilité de la prise et du durcissement et à un biomatériau plus cohérent (homogénéité du produit, temps de prise constant, absence de délitement).

Dans ce mode de mise en oeuvre, le méthicone est de préférence, préalablement solubilisé dans un solvant, puis la phase liquide ainsi obtenue est mélangée au ciment pulvérulent, et le solvant est ensuite amené à s'évaporer pour obtenir un ciment pulvérulent additivé de diméthicone.

En pratique, dans le domaine chirurgical ou dentaire, le matériau pâteux peut être fabriqué à partir d'un set mis à la disposition des praticiens, comprenant, dans deux conteneurs séparés, d'une part, une dose de ciment pulvérulent de phosphate tricalcique, phosphate tétracalcique et glycérophosphate, et de méthicone, d'autre part, une dose de solution aqueuse d'acide phosphorique et de chaux, le méthicone contenu dans la dose de ciment pulvérulent se présentant sous forme de poudre dans une proportion pondérale notamment comprise entre 0,3 % et 2 % (par rapport à ladite dose de ciment pulvérulent). Bien entendu les conteneurs contenant les dose de ciment et dose de solution sont stérilisés après scellement.

Au moment de l'intervention, le praticien ouvre les conteneurs, mélange la dose de ciment pulvérulent et la dose de solution aqueuse, homogénéise le mélange jusqu'à obtenir une pâte et, avant son évolution, injecte celle-ci dans un conduit vers le site d'implantation au moyen d'un matériel adapté (cathéter, pompe, seringue ...).

La dose de ciment et la dose de solution aqueuse sont avantageusement réalisées avec les conditions ci-dessous définies qui conduisent à un compromis particulièrement favorable du matériau pâteux injectable pour les applications médicales (composition de l'hydroxyapatite obtenue très proche de celle de la partie minérale de l'os et de la dent, temps de prise bien adapté à l'intervention -de l'ordre de 30 minutes-, absence totale de délitement, bonnes propriétés mécaniques du biomatériau obtenu...) :
- rapport atomique Ca/P compris entre 1,60 et 1,64,
- proportion pondérale de glycérophosphate comprise entre 6 % et 9 %,
- proportion pondérale de méthicone comprise entre 0,5 % et 1,2 %,
- rapport pondéral global liquide/solide compris entre 0,40 et 0,50,
   (les rapports et proportions sus-indiqués se référant au mélange pâteux final).

L'invention est illustrée, à titre non limitatif, par les exemples qui suivent, en référence aux dessins annexés, sur lesquels :
- la figure 1 est un schéma d'un dispositif permettant de mesurer une pression d'injection d'un mélange pâteux afin de définir son inj ectabilité,
- la figure 2 est une reproduction de diagrammes de diffraction infrarouge du produit obtenu à l'exemple 1 au cours de son évolution (50 minutes, 60 minutes, 24 heures après gâchage),
- la figure 3, obtenue à l'exemple 1, représente la courbe d'évolution de la résistance à la pénétration du produit en fonction du temps.

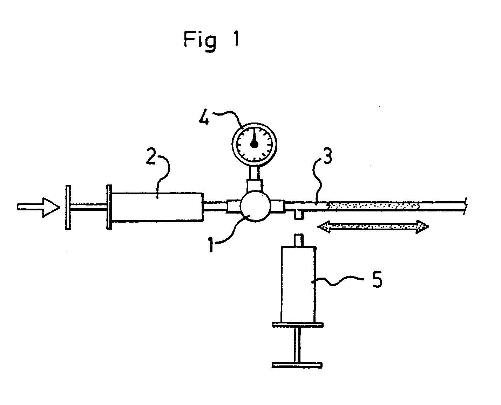

Pour évaluer l'injectabilité des mélanges pâteux préparés au cours des exemples, on utilise un système tel que représenté à la figure 1 permettant d'exercer et de mesurer une pression d'injection. Ce système se compose d'un Té de dérivation 1, d'une seringue 2, d'un cathéter 3, d'un manomètre 4 et d'une seringue séparée 5 de mise en place de matériau pâteux dans le cathéter 3 (temporairement séparé du Té de dérivation).

Chaque série de tests est effectuée en utilisant des quantités de matériau pâteux identiques mais à des instants différents après la fin du gâchage (3 min, 6 min, 10 min, 14 min, etc...).

Chaque quantité de matériau est injectée sous forme d'une colonne de 8 cm de matériau pâteux dans le cathéter 3 et on mesure pour chaque test la pression (dite pression d'injection) nécessaire pour déplacer cette colonne.

### EXEMPLES

### Exemple 1 : Préparation d'un matériau pâteux injectable Ca/P=1,634 ; L/S=6,43 ; glycérophosphate de sodium (NaGP)= 6,3 % ; diméthicone V50=0,7 %.

Le diméthicone V50 utilisé dans les exemples 1,2 et 4 est un composé cyclique de densité égale à 0,92 et de viscosité égale à 0,05 mPas (50 centistokes)
a) On prépare un mélange de poudres par pesées exactes comprenant les constituants suivants :
   - phosphate tétracalcique = 51,75 g,
   - phosphate tricalcique α = 38,25 g,
   - glycérophosphate de sodium = 9 g.

   Le rapport atomique calcium/phosphore (Ca/P) de ce mélange, hors glycérophosphate de sodium, est égal à 1,77. Ce mélange est homogénéisé d'abord à l'aide d'un mortier puis au moyen d'un mélangeur de poudre. On rajoute ensuite une solution de diméthicone (1g) préalablement solubilisée dans une petite quantité de cyclohexane (10 millilitres). Le tout est mélangé pendant une demi-heure au moyen d'un mélangeur de poudre. Cette phase solide est placée ensuite dans un cristallisoir pendant 72 heures afin de laisser évaporer le solvant
b) On prépare la solution aqueuse de phosphate et de calcium de la façon suivante :
   On introduit 6,25 g d'acide phosphorique (masse volumique = 1,69g/cm³) dans une petite quantité d'eau distillée, puis on ajoute lentement 1,646 g d'hydroxyde de calcium On complète la solution à 50 ml avec de l'eau distillée. on obtient une solution limpide, stable de rapport atomique Ca/P = 0,349.
c) On verse dans un petit mortier une quantité de 7,00 g du mélange de poudres. On ajoute ensuite une quantité de 3,01g de la solution en malaxant énergiquement au moyen d'un pilon ou d'une spatule. Le mélange est pâteux et homogène, le temps de gâchage reste fixe et égal à 2 minutes.

Le matériau pâteux préparé selon cette méthode présente un rapport atomique global Ca/P = 1,634 et un rapport L/S = 0,43.

Ce matériau est caractérisé en ce qui concerne son injectabilité et son évolution.

### Mesure d'injectabilité :

Les mesures sont effectuées au moyen du dispositif de la figure 1. On constate que la pression d'injection reste sensiblement constante et faible (0,7 bar) pendant 11 minutes environ et que cette pression croit ensuite rapidement Le matériau est donc injectable pendant un temps de 11 minutes (temps d'injectabilité) sans que ces caractéristiques d'injectabilité varient.

### Evolution du matériau pâteux:

On prépare de la même façon que précédemment des quantités de matériau pâteux nécessaires aux tests et mesures suivants.

L'évolution du matériau pâteux d'un point de vue cristallographique peut être suivie par diffraction des rayons X. On observe la disparition progressive des phases initiales du mélange et la formation après 72 heures d'une phase apatitique cristallisée pure.

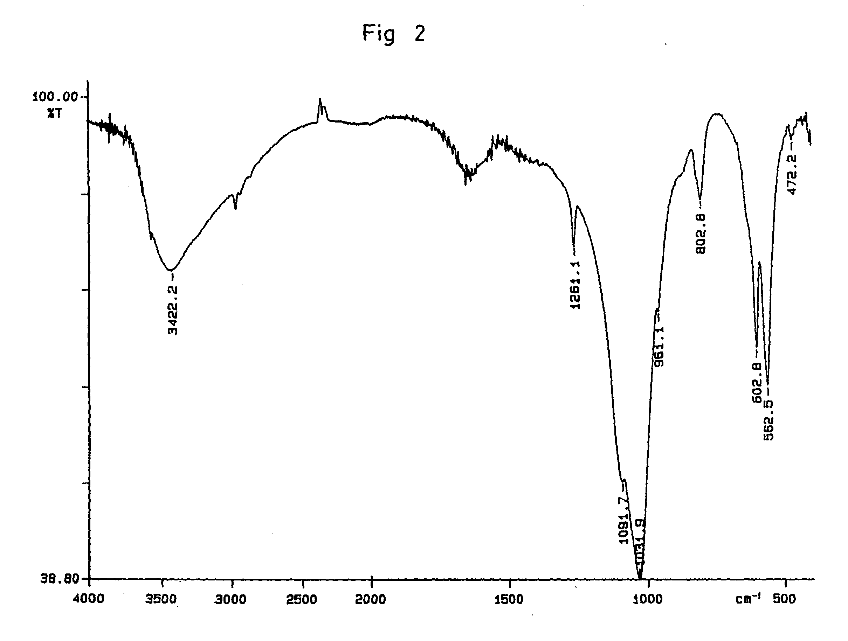

L'observation des spectres infrarouges met en évidence la présence du silicone. En effet, autour de 1260 cm⁻¹, il apparaît une bande due au silicone (figure 2).

### Temps de prise :

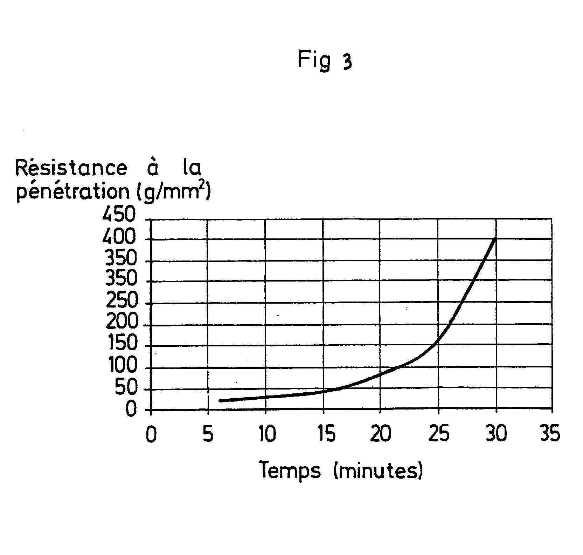

On mesure tout au long de l'évolution du mélange les modifications de la plasticité en utilisant un pénétromètre : celui-ci mesure la résistance à la pénétration à la surface du matériau pâteux d'une pointe de 1 mm². La courbe de la figure 3 donne la variation de cette résistance en fonction du temps. On peut considérer qu'à une valeur voisine de 300g/mm², le matériau a perdu toute malléabilité : il a totalement fait prise. Bien entendu, ensuite, il continue son durcissement.

Dans le cas du matériau décrit, la valeur de prise (300g/mm²) est atteinte après 28 minutes.

### Propriétés mécaniques :

On s'intéresse également aux propriétés mécaniques des biomatériaux obtenus à partir du matériau pâteux préparé. On définit la résistance à la compression sur une machine du type "Hounsfield Série S". Pour cela, on prépare cinq éprouvettes de même dimension (h = 13mm et φ = 10,6mm) que l'on laisse évoluer 7 jours à 37°C dans un milieu 100% humide.

La résistance à la compression dans cet exemple est de 25 MPa.

### Porosité:

La porosité est déterminée à partir du rapport entre la masse volumique calculée expérimentalement (masse de l'éprouvette divisée par volume de l'éprouvette) et la masse volumique théorique (masse volumique de l'hydroxyapatite = 3,15 g/cm³).

Le rapport des masses volumiques est égal à 51% donc la porosité est de 49%.

### Exemple 2 : Préparation d'un matériau pâteux injectable Ca/P=1,634 ; L/S=0,50 ; glycérophosphate de sodium (NaGP)= 6,3 % ; diméthicone V50= 0,7%.

a) On prépare le même mélange de poudres par pesées exactes, que dans l'exemple 1 (rapport Ca/P= 1,77).
b) On prépare la même solution aqueuse que dans l'exemple 1. On obtient une solution limpide, stable de rapport atomique Ca/P = 0,349.
c) On verse dans un petit mortier une quantité de 7,00 g du mélange de poudres. On ajoute ensuite une quantité de 3,01 g de la solution puis 0,49 g d'eau distillée (afin d'avoir un rapport liquide sur solide égal à 0,50) en malaxant énergiquement au moyen d'un pilon ou d'une spatule. Le mélange est pâteux et homogène, le temps de gâchage resté fixe et égal à 2 minutes.

Le matériau préparé présente un rapport atomique global Ca/P = 1,634 et un rapport L/S= 0,50.

La pression d'injection mesurée dans cet exemple est de 0,4 bar et le temps d'injection est égal à 9 minutes.

Dans le cas du matériau décrit, la valeur de prise (300 g/mm²) est atteinte après 36 minutes. La résistance à la compression du biomatériau est de 15 MPa et la porosité de 44 %.

### Exemple 3 : Préparation d'un matériau pâteux injectable Ca/P=1,634 ; L/S=0,43 ; glycérophosphate de sodium (NaGP)= 6,3 % ; diméthicone V350=0,7%.

Le diméthicone utilisé à cet exemple est un composé cyclique, de viscosité égale à 350 centistokes, ce qui correspond à une densité égale à 0,96.
a) On prépare le même mélange de poudres par pesées exactes que dans l'exemple 1 (rapport Ca/P =1,77).
b) on préparé la même solution aqueuse que dans l'exemple 1.

On obtient une solution limpide; stable, de rapport atomique Ca/P =0,349.

Le matériau préparé selon cette méthode présente un rapport atomique global Ca/P =1,634 et un rapport L/S = 0,43.

Comme dans l'exemple 1, on utilise les mêmes quantités pour déterminer les caractéristiques du matériau.

La pression d'injection mesurée dans cet exemple est de 1,1 bar et le temps d'injection est égal à 8 minutes.

Dans le cas du matériau ici décrit, la valeur de prise (300g/mm²) est atteinte après 43 minutes. La résistance à la compression du biomatériau est de 25 MPa et la porosité de 47%.

### Exemple 4 : Préparation d'un matériau pâteux injectable Ca/P=1,634; L/S=0,43 ; glycérophosphate de sodium (NaGP)= 7,0 % ; diméthicone V50=1,75%.

a) On prépare un mélange de poudres par pesées exactes comprenant les constituants suivants :
   - phosphate tétracalcique = 50,85 g
   - phosphate tricalcique α = 36,65 g
   - glycérophosphate de sodium = 10 g.

   Le rapport atomique calcium/phosphore (Ca/P) de ce mélange hors glycérophosphate de sodium est égal à 1,77. Ce mélange est homogénéisé d'abord à l'aide d'un mortier puis au moyen d'un mélangeur de poudre.
   On rajoute ensuite la solution de silicone (2,5 g) préalablement solubilisée dans une petite quantité de cyclohexane. Le tout est mélange pendant une demi-heure au moyen d'un mélangeur de poudre. Cette phase solide est placée ensuite dans un cristallisoir pendant 72 heures afin de laisser évaporer le solvant.
b) On prépare la solution aqueuse de phosphate et de calcium de la façon suivante :

On introduit 6,124 g d'acide phosphorique (masse volumique = 1,69g/cm³) dans une petite quantité d'eau distillée, puis on ajoute lentement 1,578 g d'hydroxyde de calcium. On complète la solution à 50 ml avec de l'eau distillée. On obtient une solution limpide, stable, de rapport atomique Ca/P = 0,341.

Le matériau préparé selon cette méthode présente un rapport atomique global Ca/P = 1,63 et un rapport L/S = 0,43.

Comme dans l'exemple 1, on utilise les mêmes quantités pour déterminer les caractéristiques du matériau.

La pression d'injection mesurée dans cet exemple est de 0,8 bar et le temps d'injection est égal à 7 minutes.

Dans le cas du matériau décrit, la valeur de prise (300g/mm²) est atteinte après 37 minutes. La résistance à la compression du biomatériau est de 20 MPa et la porosité de 48 %.

## Revendications

1. - Procédé de préparation d'un matériau pâteux phosphocalcique injectable appelé après injection et durcissement à former un biomatériau apatitique, dans lequel on réalise à partir d'eau et de phosphates calciques un mélange pâteux apte à évoluer, durcir et former une hydroxyapatite, **caractérisé en ce qu'**on ajoute aux phosphates calciques ou au mélange pâteux avant injection un méthicone en proportion pondérale par rapport au mélange supérieure à 0,30 % et inférieure à 10 %.

2. - Procédé de préparation selon la revendication 1, dans lequel on réalise un mélange pâteux de phosphates calciques ayant un rapport atomique Ca/P compris entre 1,5 et 1,67.

3. - Procédé de préparation selon l'une des revendications 1 ou 2, dans lequel on ajoute au mélange pâteux un glycérophosphate soluble dans l'eau, dans une proportion pondérale par rapport au mélange inférieure à 10 %.

4. - Procédé de préparation selon l'une des revendications 1, 2 ou 3, **caractérisé en ce qu'**on utilise un méthicone de viscosité sensiblement comprise entre 0,02 et 0,05 mPas (20 et 500 centistokes).

5. - Procédé de préparation selon l'une des revendications 1, 2 ou 3, **caractérisé en ce qu'**on utilise un diméthicone comportant deux groupements CH₃ sur le silicium de son motif.

6. - Procédé de préparation selon l'une des revendications 1, 2, 3, 4 ou 5, **caractérisé en ce qu'**on ajoute une proportion pondérale de méthicone sensiblement comprise entre 0,5 % et 1,4 %.

7. - Procédé de préparation selon l'une des revendications précédentes, dans lequel le mélange pâteux est réalisé à froid au moyen d'un ciment pulvérulent de phosphate tricalcique et phosphate tétracalcique, et d'une solution aqueuse contenant du calcium et du phosphate mélangés à température ambiante.

8. - Procédé de préparation selon la revendication 7, dans lequel le mélange pâteux est réalisé :
• en préparant un ciment pulvérulent par mélange de poudres de phosphate tricalcique, de phosphate tétracalcique et de glycérophosphate,
• en préparant une solution aqueuse d'acide phosphorique et de chaux,
• et en mélangeant ladite solution aqueuse et ledit ciment pulvérulent de façon que le rapport pondéral global liquide/solide soit compris entre 0,30 et 0,65, afin d'obtenir une pâte homogène de rapport atomique global Ca/P compris entre 1,50 et 1,67.

9. - Procédé de préparation selon l'une des revendications 7 ou 8, **caractérisé en ce que** l'on solubilise préalablement le méthicone dans un solvant, on mélange la phase liquide obtenue au ciment pulvérulent et on laisse évaporer le solvant.

10. - Procédé selon la revendication 9 pour la préparation d'un matériau pâteux appelé à être injecté lors d'une intervention chirurgicale ou dentaire en vue de durcir in situ après son injection sur le site d'implantation, **caractérisé en ce que** :
• on prépare préalablement une dose de ciment pulvérulent de phosphate tricalcique, phosphate tétracalcique et glycérophosphate, et méthicone,
• on prépare préalablement une dose de solution aqueuse d'acide phosphorique et de chaux,
• au moment de l'intervention, on mélange lesdites dose de ciment et dose de solution aqueuse, et, avant son évolution, on injecte le mélange dans un conduit, en particulier conduit souple de type cathéter.

11. - Procédé selon la revendication 10, **caractérisé en ce que** la dose de ciment et la dose de solution aqueuse sont préparées de façon que le rapport atomique Ca/P du mélange final soit compris entre 1,60 et 1,64, que la proportion pondérale de glycérophosphate par rapport au mélange final soit comprise entre 6 % et 9 %, que la proportion pondérale de méthicone par rapport au mélange final soit comprise entre 0,5 % et 1,2 %, et que le rapport pondéral global liquide/solide soit compris entre 0,40 et 0,50.

12. - Set chirurgical ou dentaire comprenant dans deux conteneurs séparés, d'une part, une dose de ciment pulvérulent de phosphate tricalcique, phosphate tétracalcique, glycérophosphate, d'autre part, une dose de solution aqueuse d'acide phosphorique et de chaux, **caractérisé en ce que** la dose de ciment pulvérulent contient un méthicone en proportion pondérale comprise entre 0,3 % et 2 % par rapport à ladite dose.

## Claims

1. Process for the preparation of an injectable pasty calcium phosphate material, which, after injection and hardening, is capable of forming an apatite biomaterial, wherein water and calcium phosphates are used to produce a pasty mixture that is capable of developing, hardening and forming a hydroxyapatite, **characterised in that**, before injection, a methicone is added to the calcium phosphates or to the pasty mixture in a proportion by weight in comparison to the mixture that is greater than 0.30% and less than 10%.

2. Process of preparation according to claim 1, wherein a pasty mixture of calcium phosphates is produced with an atomic ratio Ca/P of 1.5 to 1.67.

3. Process of preparation according to one of claims 1 or 2, wherein a water-soluble glycerophosphate is added to the pasty mixture in a proportion by weight in comparison to the mixture that is less than 10%.

4. Process of preparation according to one of claims 1, 2 or 3, **characterised in that** a methicone with viscosity from 0.02 to 0.05 mPas (20 to 500 centistokes) is used.

5. Process of preparation according to one of claims 1, 2 or 3, **characterised in that** a dimethicone that includes two CH₃ groups in the silicon of its structure is used.

6. Process of preparation according to one of claims 1, 2, 3, 4 or 5, **characterised in that** a proportion by weight of methicone of approximately 0.5% to 1.4% is added.

7. Process of preparation according to one of the preceding claims, wherein the pasty mixture is produced cold by means of a powdered cement of tricalcium phosphate and tetracalcium phosphate, and by an aqueous solution containing calcium and phosphate mixed at room temperature.

8. Process of preparation according to claim 7, wherein the pasty mixture is produced:
• by preparing a powdered cement by mixing powders of tricalcium phosphate, tetracalcium phosphate and glycerophosphate,
• by preparing an aqueous solution of phosphoric acid and lime,
• and by mixing the said aqueous solution and the said powdered cement such that the overall weight ratio of liquid to solid is from 0.30 to 0.65 so as to obtain a homogeneous paste with an overall atomic ratio Ca/P of 1.50 to 1.67.

9. Process of preparation according to one of claims 7 or 8, **characterised in that** the methicone is solubilised beforehand in a solvent and the liquid phase obtained is then mixed with the powdered cement and the solvent is allowed to evaporate.

10. Process of preparation according to claim 9 for the preparation of a pasty material to be injected during a surgical or dental procedure with a view to it hardening in situ after injection at the site of implantation,
**characterised in that**:
• a dose of powdered cement of tricalcium phosphate, tetracalcium phosphate and glycerophosphate, and methicone is prepared beforehand,
• a dose of aqueous solution of phosphoric acid and lime is prepared beforehand,
• at the moment of the procedure, the said dose of cement and dose of aqueous solution are mixed, and, before it develops, the mixture is injected into a tube, in particular a flexible tube of the catheter type.

11. Process of preparation according to claim 10, **characterised in that** the dose of cement and the dose of aqueous solution are prepared such that the atomic ratio Ca/P of the final mixture is from 1.60 to 1.64, **in that** the proportion by weight of glycerophosphate in comparison to the final mixture is from 6% to 9%, **in that** the proportion by weight of methicone in comparison to the final mixture is from 0.5% to 1.2% and **in that** the overall weight ratio of liquid to solid is from 0.40 to 0.50.

12. Surgical or dental kit comprising in two separate containers, on the one hand a dose of powdered cement of tricalcium phosphate, tetracalcium phosphate and glycerophosphate, and on the other hand, a dose of aqueous solution of phosphoric acid and lime, **characterised in that** the dose of powdered cement contains a methicone in a proportion by weight from 0.3% to 2% in comparison to the said dose.

## Patentansprüche

1. Verfahren zur Herstellung eines injizierbaren pastösen Calciumphosphat enthaltenden Materials, das nach Injektion und Erhärtung zum Bilden eines apatitischen Biomaterials bestimmt ist, bis dem ausgehend von Wasser und Calciumphosphaten eine pastöse Mischung hergestellt wird, die weiter verändert und erhärtet werden kann und ein Hydroxyapatit bilden kann,
**dadurch gekennzeichnet,**
**dass** zu den Calciumphosphaten oder zu der pastösen Mischung vor einer Injektion ein Methicon in einem Gewichtsverhältnis in Bezug auf die Mischung von mehr als 0,30 % und weniger als 10 % hinzugefügt wird.

2. Verfahren zur Herstellung nach Anspruch 1, bei dem eine pastöse Mischung von Calciumphosphaten mit einem atomaren Verhältnis Ca/P zwischen 1,5 und 1,67 hergestellt wird.

3. Verfahren zur Herstellung nach einem der Ansprüche 1 oder 2, bei dem zu der pastösen Mischung ein in Wasser lösliches Glycerolphosphat in einem Gewichtsverhältnis in Bezug auf die Mischung von weniger als 10 % hinzugegeben wird.

4. Verfahren zur Herstellung nach einem der Ansprüche 1, 2 oder 3,
**dadurch gekennzeichnet,**
**dass** ein Methicon mit einer Viskosität, die im Wesentlichen zwischen 0,02 und 0,05 mPas (20 und 50 centistokes) liegt, verwendet wird.

5. Verfahren zur Herstellung nach einem der Ansprüche 1, 2 oder 3,
**dadurch gekennzeichnet,**
**dass** ein Dimethicon verwendet wird, das zwei CH₃-Gruppen an dem Silizium seiner Grundeinheit aufweist.

6. Verfahren zur Herstellung nach einem der Ansprüche 1, 2, 3, 4 oder 5,
**dadurch gekennzeichnet, dass** ein Gewichtsverhältnis an Methicon hinzugegeben wird, das im Wesentlichen zwischen 0,5 % und 1,4 % liegt.

7. Verfahren zur Herstellung nach einem der vorhergehenden Ansprüche, bei dem die pastöse Mischung auf kaltem Wege mit Hilfe eines pulverförmigen Zements von Tricalciumphosphat und Tetracalciumphosphat sowie einer wässrigen Lösung, die bei Raumtemperatur vermischtes Calcium und Phosphat enthält, hergestellt wird.

8. Verfahren zur Herstellung nach Anspruch 7, bei dem die pastöse Mischung hergestellt wird:
- durch Herstellen eines pulverförmigen Zements durch Mischen von Pulver von Tricalciumphosphat, Tetracalciumphosphat und Glycerolphosphat,
- durch Herstellen einer wässrigen Lösung von Phosphorsäure und Kalk,
- und durch Vermischen der wässrigen Mischung und des pulverförmigen Zements auf eine Weise, so dass das gesamte flüssig/fest Gewichtsverhältnis zwischen 0,30 und 0,65 liegt, um eine homogene Paste mit einem atomaren Gesamtverhältnis Ca/P, das zwischen 1,50 und 1,67 liegt, zu erhalten.

9. Verfahren zur Herstellung nach einem der Ansprüche 7 oder 8,
**dadurch gekennzeichnet,**
**dass** zunächst das Methicon in einem Lösungsmittel gelöst wird, und die erhaltene flüssige Phase mit dem pulverförmigen Zement vermischt wird, und das Lösungsmittel verdampft wird.

10. Verfahren nach Anspruch 9 zur Herstellung eines pastösen Materials, das dazu vorgesehen ist, während eines chirurgischen oder zahnärztlichen Eingriffs nach seiner Injektion an der Stelle der Implantation im Hinblick auf eine in-situ-Erhärtung eingespritzt zu werden,
**dadurch gekennzeichnet,**
**dass**:
- vorher eine Dosis eines pulverförmigen Zements aus Tricalciumphosphat, Tetracalciumphosphat und Glycerolphosphat sowie Methicon hergestellt wird,
- vorher eine Dosis einer wässrigen Lösung von Phosphorsäure und Kalk hergestellt wird,
- zum Zeitpunkt des Eingriffs die Dosis an Zement und die Dosis der wässrigen Lösung vermischt werden, und die Mischung vor ihrer Veränderung in eine Leitung, insbesondere eine biegsame Leitung von der Art eines Katheters, injiziert wird.

11. Verfahren nach Anspruch 10,
**dadurch gekennzeichnet,**
**dass** die Dosis an Zement und die Dosis der wässrigen Lösung auf eine Weise hergestellt werden, so dass das atomare Verhältnis Ca/P der endgültigen Mischung zwischen 1,60 und 1,64 liegt,
**dass** das Gewichtsverhältnis des Glycerolphosphats in Bezug auf die endgültige Mischung zwischen 6 % und 9 % liegt,
**dass** das Gewichtsverhältnis von Methicon in Bezug auf die endgültige Mischung zwischen 0,5 % und 1,2 % liegt, und
**dass** das gesamte flüssig/fest Gewichtsverhältnis zwischen 0,40 und 0,50 liegt.

12. Chirurgisches oder zahnärztliches Set, das in zwei getrennten Behältern enthalten ist, einerseits einer Dosis eines pulverförmigen Zements von Tricalciumphosphat, Tetracalciumphosphat, Glycerolphosphat, und andererseits einer Dosis einer wässrigen Lösung von Phosphorsäure und Kalk,
**dadurch gekennzeichnet,**
**dass** die Dosis des pulverförmigen Zements ein Methicon enthält, in einem Gewichtsverhältnis, das in Bezug auf die Dosis zwischen 0,3 % und 2 % liegt.
